# EUROPÄISCHE PATENTSCHRIFT

(11) **EP 0 346 852 B1**
(45) Veröffentlichungstag und Bekanntmachung des Hinweises auf die Patenterteilung: **23.02.1994**
(21) Anmeldenummer: 89110723.7
(22) Anmeldetag: 13.06.1989
(51) Int. Cl.: C07C 69/734, C07C 67/327, C07C 67/31, C07C 69/708

(54) **Verfahren zur Herstellung von 4-chlor-3-alkoxy-but-2E-en-säurealkylester**
Process for producing 4-chloro-3-alkoxybut-2E-ene acid alkyl esters
Procédé de préparation d'esters de l'acide 4-chloro-3-alcoxy-but-3E-énoique

(30) Priorität: 14.06.1988 CH 2288/88
(43) Veröffentlichungstag der Anmeldung: 20.12.1989
(73) Patentinhaber: LONZA AG, CH-4002 Basel (CH)
(72) Erfinder: Duc, Laurent, Dr., Sion (Kanton Wallis) (CH)
(74) Vertreter: Weinhold, Peter, Dr.

(56) Entgegenhaltungen:
- EP-A- 0 216 324

## Beschreibung

Gegenstand der Erfindung ist ein neues Verfahren zur Herstellung der im Titel genannten Verbindungen.

Diese Verbindungen sind vielseitige Bausteine für zahlreiche Synthesen von Wirkstoffen für u.a. Pharmazeutika, Agrochemikalien etc.

Beispielsweise finden diese Zwischenprodukte Einsatz in der Herstellung von 4-Hydroxy-2-oxo-pyrrolidin-1-yl-acetamid, einem cerebral wirksamen Pharmazeutika (EP-A 0 216 324).

Aus der genannten EP-A 0 216 324 geht hervor, dass die 4-Chlor-3-alkoxy-but-2E-en-säurealkylester durch Umsetzung von 4-Chloracetessigsäurealkylester mit einem Orthoameisensäuretrialkylester in Gegenwart von Schwefelsäure zum Ketalester und Erhitzen in Vacuum hergestellt werden können. Zwar sind nach diesen bekannten Verfahren gute Ausbeuten von 90 bis 93% möglich, nachteilig ist aber einerseits die ungenügende Reinheit der erhaltenen Produkte, anderseits wirkt sich der hohe Preis des Orthoameisensäuretrialkylesters belastend auf die Gestehungskosten einer mehrstufigen Wirkstoffsynthese aus. Vom Sicherheitsaspekt her gesehen nicht unbedenklich ist ausserdem die Bildung des äusserst giftigen Dimethylsulfats bei der Reaktion von Othoameisensäuretrialkylester mit Schwefelsäure.

Es stellte sich daher die Aufgabe, ein Verfahren zu finden, das die genannten Nachteile nicht aufweist und mit dem man in der Lage ist, 4-Chlor-3-alkoxy-but-2E-en-säurealkylester in grosstechnischem Massstab kostengünstig und sicherheitstechnisch unbedenklich herzustellen.

Diese Aufgabe konnte mit einem Verfahren nach Patentanspruch 1 gelöst werden.

Ausgangsprodukt des Verfahrens ist das grosstechnisch aus Diketen und Chlor hergestellte 4-Chloracetessigsäurechlorid, vorzugsweise gelöst in Methylenchlorid vorliegend.

Die Umsetzung des 4-Chloracetessigsäurechlorids zum Ketalester der Formel 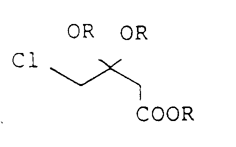
worin R Alkyl mit 1 bis 4 C-Atomen bedeutet, erfolgt darauf mit in situ erzeugtem Dialkylsulfit der Formel

(RO)₂S = O

und dem entsprechenden Alkohol ROH. In situ erzeugtes Dialkylsulfit bedeutet, dass durch Zugabe von Thionylchlorid und dem entsprechenden Alkohol zum Reaktionsgemisch die notwendige Menge Dialkylsulfit generiert wird und unmittelbar für die Ketalbildung zur Verfügung steht. Eine zusätzliche Menge des entsprechenden Alkohols ROH ist notwendig, um das Säurechlorid in den Ester umzuwandeln. Zweckmässig wird vom Alkohol ROH daher ein Ueberschuss eingesetzt. Die Gesamtmenge Alkohol bewegt sich zweckmässig zwischen 5 und 20 Mol pro Mol 4-Chloracetessigsäurechlorid. Als geeignete Alkohole ROH werden im Hinblick auf den Substituenten R im Endprodukt und dessen Weiterverwendung von allem die niederen aliphatischen Alkohole,wie Methanol, Ethanol, Propanol und Butanol eingesetzt.

Da das 4-Chloracetessigsäurechlorid in der Regel direkt dem Herstellungsprozess (aus Diketen und Chlor) entnommen wird, liegt es in Form einer Lösung, zweckmässig in einem inerten Lösungsmittel wie Methylenchlorid, vor.

Es ist ein weiterer Vorzug des erfindungsgemässen Verfahrens, dass für die Umsetzung zum Ketalester direkt diese Lösung von 4-Chloracetessigsäurechlorid eingesetzt werden kann. Die Umsetzung zum Ketalester wird zweckmässig bei Temperaturen zwischen -10 und 120°C, vorzugsweise bei Raumtemperatur durchgeführt. Erfahrungsgemäss kann mit einer Reaktionszeit von 2 bis 5 Stunden gerechnet werden. Zwar kann der Ketalester isoliert werden, vorteilhaft wird aber direkt zum gewünschten Endprodukt weiterumgesetzt.

Dazu wird das Reaktionsgemisch, zweckmässig nach vorherigem Entfernen der Lösungsmittel, mit einer Säure versetzt und durch Erwärmen bei reduziertem Druck in das Endprodukt überführt. Als Säure finden zweckmässig Schwefelsäuren oder eine Sulfonsäure, wie Methansulfonsäure oder p-Toluolsulfonsäure in katalytischen Mengen von 0,4 bis 1 Mol% Anwendung.

Die Temperaturen für die Umwandlung des Ketalesters in das Endprodukt liegen zweckmässig zwischen 70 und 150°C, vorzugsweise zwischen 100 und 130°C. Dabei wird ein reduzierter Druck zwischen 50 und 500 mbar, vorteilhaft zwischen 75 und 100 mbar aufrecht erhalten.

Der resultierende 4-Chlor-3-alkoxy-but-2E-en-säurealkylester kann nach dieser Behandlung auf übliche Art und Weise isoliert und gegebenenfalls gereinigt werden.

Nach dem erfindungsgemässen Verfahren lassen sich Ausbeuten von gegen 90% und Reinheiten der Produkte von grösser als 99% erzielen.

### Beispiel 1

### 4-Chlor-3-methoxybut-2E-en-säuremethylester

206,6 g (0,47 Mol) einer 35%igen Mischung von 4-Chloracetoacetylchlorid in Methylenchlorid wurden auf -10°C abgekühlt.

Unter Stickstoff wurden während 30 Minuten 102,4 g (3,2 Mol) Methanol dann wiederum während 30 Minuten 83,3 g (0,7 Mol) Thionylchlorid zugegeben (Bildung von Dimethylsulfit). Man erhöhte die Temperatur auf Raumtemperatur und liess die Lösung während 3 Stunden bei 20 bis 25°C rühren. Das überschüssige Methanol und Methylenchlorid wurde darauf bei reduziertem Druck abdestilliert. Der Rückstand (roher 4-Chlor-3,3-dimethoxybutansäuremethylester) wurde mit 0,21 g Methansulfonsäure versetzt und bei einem Druck von 100 mbar auf 125 bis 130°C erwärmt. Dabei wurde das gebildete Methanol und der Ueberschuss Dimethylsulfit abdestilliert. Der Rückstand (roher 4-Chlor-3-methoxy-but-2E-en-säuremethylester) wurde aufgenommen in 110,2 g (120 ml) Toluol und die organische Phase wurde gewaschen mit 69,8 g einer wässrigen 16% HCl während 30 Minuten, mit 32,1 g einer wässrigen 10% Kochsalzlösung während 10 Minuten, mit 134 g einer wässrigen 10% NaOH während 75 Minuten und schliesslich mit 32,1 g einer wässrigen 10% Kochsalzlösung während 10 Minuten. Das Toluol wurde daraufhin abgedampft und der Rückstand bei einem Druck von 20 mbar und bei einer Temperatur von 95 bis 97°C destilliert.

Das Titelprodukt wurde in einer Ausbeute von 61,7 g (80%) in einer Reinheit von 99,5% (GC) erhalten.

### Beispiel 2

### 4-Chlor-3-ethoxybut-2E-en-säureethylester

206,6 g (0,47 Mol) einer 35%igen Mischung von 4-Chloracetoacetylchlorid in Methylenchlorid wurde auf -10°C abgekühlt.

Unter Stickstoff wurden während 30 Minuten 147,2 g (3,2 Mol) Ethanol dann wiederum während 30 Minuten 83,3 g (0,7 Mol) Thionylchlorid zugegeben (Bildung von Diethylsulfit). Man erhöhte die Temperatur auf 55 bis 60°C während 1 Stunde, dann liess man bei 55 bis 60°C zusätzlich 1 Stunde nachrühren. Das überschüssige Ethanol und Methylenchlorid wurde darauf bei reduziertem Druck abdestilliert. Der Rückstand (roher 4-Chlor-3,3-diethoxybutansäureethylester) wurde mit 0,27 g Methansulfonsäure versetzt und bei einem Druck von 75 mbar auf 125 bis 130°C erwärmt. Dabei wurde das gebildete Ethanol und der Ueberschuss Diethylsulfit abdestilliert. Der Rückstand (roher 4-Chlor-3-ethoxy-but-2E-en-säureethylester) wurde aufgenommen in 110,2 g (120 ml) Toluol und die organische Phase wurde gewaschen mit 69,8 g einer wässrigen 16% HCl während 30 Minuten, mit 32,1 g einer wässrigen 10% Kochsalzlösung während 10 Minuten, mit 134 g einer wässrigen 10% NaOH während 75 Minuten und schliesslich mit 32,1 g einer wässrigen 10% Kochsalzlösung während 10 Minuten. Das Toluol wurde daraufhin abgedampft und der Rückstand bei einem Druck von 2 mbar und bei einer Temperatur von 78 bis 80°C destilliert.

Das Titelprodukt wurde in einer Ausbeute von 80,6 g (88,5%) in einer Reinheit von 99,8% (GC) erhalten.

## Patentansprüche

1. Verfahren zur Herstellung von 4-Chlor-3-alkoxy-but-2E-en-säurealkylester der Formel 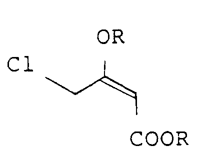 worin R Alkyl mit 1 bis 4 C-Atomen bedeutet, dadurch gekennzeichnet, dass man 4-Chloracetessigsäurechlorid mit einem Dialkylsulfit der Formel
(RO)₂S = O
und dem entsprechenden Alkohol ROH zum Ketalester der Formel 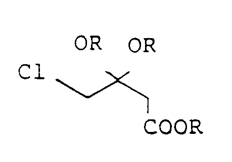 umsetzt und diesen Ketalester ohne zu isolieren durch Erwärmen unter reduziertem Druck in Gegenwart einer Säure zum Endprodukt umsetzt.

2. Verfahren nach Patentanspruch 1, dadurch gekennzeichnet, dass das Dialkylsulfit in situ durch Umsetzung von Thionylchlorid mit dem entsprechenden Alkohol ROH hergestellt worden ist.

3. Verfahren nach einem oder beiden der Patentansprüche 1-2, dadurch gekennzeichnet, dass der Alkohol für die in situ Dialkylsulfitbildung und der Alkohol für die Esterbildung gleichzeitig in einer Menge von 5 bis 20 Mol, bezogen auf 1 Mol 4-Chloracetessigsäurechlorid, zugegeben wird.

4. Verfahren nach mindestens einem der Patentansprüche 1, 2 und 3, dadurch gekennzeichnet, dass die Umsetzung zum Ketalester bei Temperaturen von -10 bis 120°C erfolgt.

5. Verfahren nach mindestens einem der Patentansprüche 1,2, 3 und 4, dadurch gekennzeichnet, dass die Umsetzung zum Ketalester in Gegenwart eines inerten Lösungsmittels erfolgt.

6. Verfahren nach mindestens einem der Patentansprüche 1,2,3, 4 und 5, dadurch gekennzeichnet, dass das gegebenenfalls in situ erzeugte Dialkylsulfit in einem Molverhältnis von 2 zu 1 und 1,4 zu 1 zum 4-Chloracetessigsäurechlorid vorliegt.

7. Verfahren nach Patentanspruch 1, dadurch gekennzeichnet, dass die Ueberführung des Ketalesters in das Endprodukt bei Temperaturen zwischen 70 und 150°C und einem Druck zwischen 50 und 500 mbar erfolgt.

8. Verfahren nach Patentansprüchen 1 und 7, dadurch gekennzeichnet, dass die Ueberführung des Ketalesters in das Endprodukt in Gegenwart einer katalytischen Menge von 0,4 bis 1 Mol% von Schwefelsäure oder einer Sulfonsäure erfolgt.

## Claims

1. A process for producing 4-chloro-3-alkoxy-but-2E-enoic acid alkyl ester of the formula: 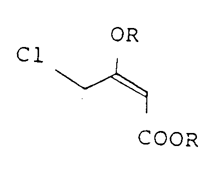 wherein R is alkyl having 1 to 4 carbon atoms;
characterized by reacting 4-chloroacetoacetic acid chloride with a dialkyl sulfite of the formula:
(RO)₂S=O
and with the corresponding alcohol ROH to form a ketal ester of the formula: 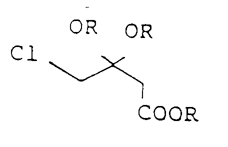 and converting said ketal ester without isolation into the final product by heating under reduced pressure in the presence of an acid.

2. The process according to Claim 1 , characterized in that the dialkyl sulfite is produced in situ by reaction of thionyl chloride with the corresponding alcohol ROH.

3. The process according to one or both of Claims 1 - 2, characterized in that the alcohol for in-situ formation of dialkyl sulfite and the alcohol for ester formation are added simultaneously in an amount of from 5 to 20 moles based upon 1 mole of 4-chloroacetoacetic acid chloride.

4. The process according to at least one of Claims 1, 2 and 3, characterized in that conversion into the ketal ester takes place at temperatures of from -10 to +120°C.

5. The process according to at least one of Claims 1, 2, 3 and 4, characterized in that conversion into the ketal ester takes place in the presence of an inert solvent.

6. The process according to at least one of Claims 1, 2, 3, 4 and 5, characterized in that the dialkyl sulfite, optionally produced in situ, is present in a molar ratio of 2/1 and 1.4/1, relative to 4-chloroacetoacetic acid chloride.

7. The process according to Claim 1, characterized in that conversion of the ketal ester into the final product takes place at temperatures of from 70 to 150°C and a pressure of from 50 to 500 mbar.

8. The process according to Claims 1 and 7, characterized in that conversion of the ketal ester into the final product takes place in the presence of a catalytic amount of from 0.4 to 1 mole-% of sulfuric acid or of a sulfonic acid.

## Revendications

1. Procédé pour la préparation d'ester alkylique de l'acide 4-chlor-3-alcoxy-but-2E-ène de la formule 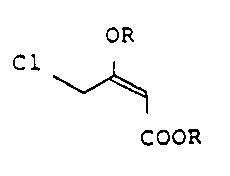 dans laquelle R signifie alkyle avec 1 jusqu'à 4 atomes C, caractérisé en ce que l'on transforme le chlorure de l'acide 4-chloroacétique avec un dialkylsulfite de la formule
(RO)₂S=O
et l'alcool correspondant ROH en cétalester de la formule 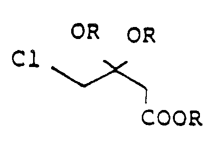 et l'on transforme ce cétalester sans isolation par chauffage sous pression réduite en présence d'un acide en produit final.

2. Procédé selon la revendication 1, caractérisé en ce que le dialkylsulfite est préparé in situ par mise en réaction de chlorure de thionyle avec l'alcool correspondant ROH.

3. Procédé selon l'une ou les deux revendications 1 et 2, caractérisé en ce que l'alcool pour la formation de dialkylsulfite in situ et l'alcool pour la formation d'ester est ajouté simultanément en une quantité de 5 à 20 moles rapportés à 1 mole de chlorure de l'acide 4-chloroacétique.

4. Procédé selon au moins l'une des revendications 1, 2 et 3, caractérisé en ce que la transformation en cétalester s'effectue à des températures de -10 jusqu'à 120°C.

5. Procédé selon au moins l'une des revendications 1, 2, 3 et 4, caractérisé en ce que la transformation en cétalester s'effectue en présence d'un solvant inerte.

6. Procédé selon au moins l'une des revendications 1, 2, 3, 4 et 5, caractérisé en ce que le dialkylsulfite produit éventuellement in situ est présent dans un rapport molaire de 2 à 1 et de 1,4 à 1 par rapport au chlorure de l'acide 4-chloroacétique.

7. Procédé selon la revendication 1, caractérisé en ce que la transformation du cétalester en produit final s'effectue à des températures entre 70 et 150°C et une pression entre 50 et 500 mbars.

8. Procédé selon les revendications 1 et 7, caractérisé en ce que la transformation du cétalester en produit final s'effectue en présence d'une quantité catalytique de 0,4 à 1 mole % d'acide sulfurique ou d'un acide sulfonique.
